# EUROPEAN PATENT APPLICATION

(11) **EP 3 187 180 A1**
(43) Date of publication of application: **05.07.2017**
(21) Application number: 15202834.6
(22) Date of filing: 28.12.2015
(51) Int. Cl.: A61K 31/122, A61K 31/7056, A61K 31/706, A61K 31/7084, A61P 43/00

(54) **ADENOSYLHOMOCYSTEINASE BINDING SUBSTANCES FOR MEDICAL USE**

(71) Applicant: Ludwig-Maximilians-Universität München, 80539 München (DE)
(72) Inventor: GERSTIN, Søren Waldemar, 80336 München (DE); MUNTAU, Ania Carolina, 20149 Hamburg (DE); ZOUBEK, Carolin Teresa Maria, 82284 Grafrath (DE)
(74) Representative: Dr. Gassner & Partner mbB

(57) **Abstract**

The invention concerns an adenosylhomocysteinase (AHCY) binding substance for use in the treatment of deficiency of function of AHCY in a human being or an animal, wherein the AHCY binding substance is the cofactor nicotinamide adenine dinucleotide (NAD⁺), or an analogue of the cofactor NAD⁺ which analogue binds to an NAD⁺ binding site of AHCY, or an analogue of a substrate of AHCY which analogue binds to a substrate binding site of AHCY.

## Description

The invention concerns an adenosylhomocysteinase (AHCY) binding substance. AHCY forms an enzymatical active tetramer. AHCY was formerly named S-adenosylhomocysteine hydrolase (SAHH). Missense mutations in the AHCY gene result in AHCY having a significantly reduced enzymatic activity. Patients with such a reduced enzymatic activity show a severe phenotype such as severe myopathy and hypotonia, developmental delay, impaired myelination and hepatopathy. An effective treatment for the disorder is not available.

From Belužić, R. and Vugrek, O., Rad 508. Medical Sciencies 35(2010), pages 77 to 92 "S-Adenosylhomocysteine Hydrolase (AHCY) Deficiency: A Natural Model System for Methylation Research" it is known that AHCY deficiency is a human methylation disorder. Cellular methylations are catalyzed by substrate-specific methyltransferase enzymes. All these enzymes share the same methyl donor, namely S-adenosylmethionine (SAM). Apart from methylated substrate, SAM-dependent methylation reactions result in formation of S-adenosylhomocysteine (SAH). SAH is hydrolyzed to adenosine and homocysteine by AHCY. While adenosine is further deaminated to inosine, homocysteine is either remethylated to methionine or enters the transsulfuration pathway resulting in formation of cystathionine or, finally, cysteine. Remethylation pathway enzymes methionine synthase and betaine-homocysteine methyltransferase together with methionine-adenosyltransferase and AHCY complete the metabolic cycling of methionine and homocysteine. SAH strongly inhibits many SAM-dependent methyltransferases. SAH hydrolysis is the only source of homocysteine in mammals. Therefore, an impaired AHCY activity would effect a wide variety of cellular processes.

It is speculated that the described mutations either result in a high instability of resulting proteins or in a steric change impairing the substrate or water molecule access or product release. The publication discloses a therapy including restricted methionine intake with supplementation of phosphatidylcholine, creatine and cysteine. However, this diet alone improved the severe phenotype only gradually.

The doctoral theses "Einfluss des Kofaktors NAD⁺/NADH der S-Adenosylhomocystein-Hydrolase auf die Adenosinbindung", Lüdtke Angelika, 2003, Faculty of Medicine of the Eberhard-Karls-Universität Tübingen, Germany, discloses the binding of the NAD⁺ molecule in a groove of the NAD⁺ binding domain of AHCY via hydrophobic interactions and hydrogen bonds. Furthermore, it is disclosed that the NAD⁺ binding domain is located in each monomer near the center of a tetramer formed from AHCY monomers and that these binding domains are interconnected which interconnection supports stability of AHCY.

Belužić, R., et al., Biochemical and Biophysical Research Communications 368 (2008), pages 30 to 36, Belužić, R., et al., Biochem. J. (2006) 400, pages 245 to 253 and Barić, I., J. Inherit. Metab. Dis. (2009) 32, pages 459 to 471 disclose R49C, D86G, A89V and Y143C mutations resulting in an exchange of amino acids in the protein. It is speculated that in case of the A89V mutation structural abnormalities represent one basis for the pathological effects and that the Y143C mutation results in a reduced stability of the tetramer resulting in a disassembly into monomers followed by a loss of enzymatic activity.

From Muntau, A. C., et al., N. Engl. J. Med., Vol. 347, No. 26, December 26, 2002 tetrahydrobiopterin is known as an alternative treatment for mild phenylketonuria. Tetrahydrobiopterin is a natural cofactor of aromatic amino acid hydroxylases and nitric oxide synthase. It is speculated that mutations in the phenylalanine hydroxylase gene may result in misfolding of the protein and reduced enzyme activity and that tetrahydrobiopterin may act as a chemical chaperone that prevents misfolding.

The problem to be solved by the present invention is to provide a substance for use in the treatment of deficiency of function of AHCY.

The problem is solved by the features of claim 1. Embodiments are disclosed in dependent claims 2 to 4.

The invention provides an AHCY binding substance for use in the treatment of deficiency of function of AHCY in a human being or an animal, wherein the AHCY binding substance is the cofactor nicotinamide adenine dinucleotide (NAD⁺), or an analogue of the cofactor NAD⁺ which analogue binds to an NAD⁺ binding site of AHCY, or an analogue of a substrate of AHCY which analogue binds to a substrate binding site of AHCY. The AHCY binding substance may be a AHCY binding molecule.

The inventors recognized that all four known missense mutations in the AHCY gene result in a reduced thermal stability and proneness of the protein to aggregate. They further recognized that the mutations neither concern the active center nor the binding sites for the substrate S-adenosyl-L-homocysteine nor for the cofactor NAD⁺. They showed in cultured cells that the conformational instability is accompanied by a loss of function of the AHCY protein.

They further recognized that the protein or the protein tetramer can be stabilized by any AHCY binding substance that binds to the binding site of the cofactor or to the substrate binding site of AHCY.

The AHCY binding substance binding to the cofactor binding site is NAD⁺ or an analogue of the cofactor NAD⁺. An analogue of NAD⁺ can be understood as any substance or molecule that binds to the NAD⁺ binding site of AHCY without being NAD⁺. The AHCY binding substance binding to the substrate binding site of AHCY is an analogue of a substrate of AHCY. An analogue of a substrate of AHCY can be understood as any substance or molecule that binds to the substrate binding site of AHCY without being a substrate of AHCY or without being the natural substrate of AHCY.

The analogue of the cofactor NAD⁺ may be thionicotinamide adenine dinucleotide (S-NAD⁺), nicotinamide 1,N⁶-ethenoadenine dinucleotide (E-NAD⁺), β-nicotinamide adenine dinucleotide (β-NAD⁺), nicotinamide hypoxanthine dinucleotide (deamino-NAD⁺), nicotinamide guanine dinucleotide, α-dihydronicotinamide adenine dinucleotide (α-NADH), β-dihydronicotinamide adenine dinucleotide (β-NADH), β-nicotinamide adenine dinucleotide phosphate (β-NADP), nicotinamide adenine triphosphate (AP3), ribofuranosyl-nicotinamide diphosphate (dAP2), ribofuranosyl-nicotinamide triphosphate (ApCpp), nicotinamide thymine dinucleotide (TP2), nicotinamide uracil dinucleotide (UP2), nicotinamide cytosine dinucleotide (CP2), nicotinamide guanine dinucleotide (GP2), nicotinamide 8-bromo-adenine dinucleotide (8-Bromo-AP2), 2'-bromo-ribofuranosyl-nicotinamide diphosphate (2'-Bromo-dAP2), P¹-(5'-adenosyl) P³-[5'-(1-β D-ribofuranosyl-nicotinamide)] triphosphate (AP3(Nic)), 1,2,4-triazole-3-carboxamide adenine dinucleotide (AP2Ribavirin), P¹-[5'-(2'-deoxy-adenosyl)] P²-[5'-(1-β D-ribofuranosyl-nicotinamide)] diphosphate (dAP2(Nic)), P¹-(5'-adenosyl) P³-[5'-(1-β D-ribofuranosyl-nicotinamide)] [(α,β)-methyleno]triphosphate (ApCpp(Nic)), nicotinamide thymine dinucleotide (TP2(Nic)), nicotinamide uracil dinucleotide (UP2(Nic)), nicotinamide cytosine dinucleotide (CP2(Nic)), nicotinamide guanine dinucleotide (GP2(Nic)), nicotinamide 8-bromo-adenine dinucleotide (8-Bromo-AP2(Nic)), an ion of any of said analogues which analogue is not charged, or a hydrate of any of said analogues.

The ion of any of said analogues may be an ion of a sodium salt.

The analogue of the substrate may be 1-[(2*R*,3*R*,4*S*,5*R*)-3,4-dihydroxy-5-(hydroxymethyl)oxolan-2-yl]-1*H*-1,2,4-triazole-3-carboxamide (ribavirin), 3-Deazaneplanocin A (DZNep) or ilimaquinone.

All the AHCY binding molecules according to the invention may act as pharmacological chaperones by stabilization of the AHCY monomer or tetramer.

### Embodiments:

- Fig. 1a - Fig. 1f: show results obtained by testing of wild-type (wt) protein and mutant proteins together with different concentrations of NAD⁺ in Differential Scanning Fluorimetry (DSF),

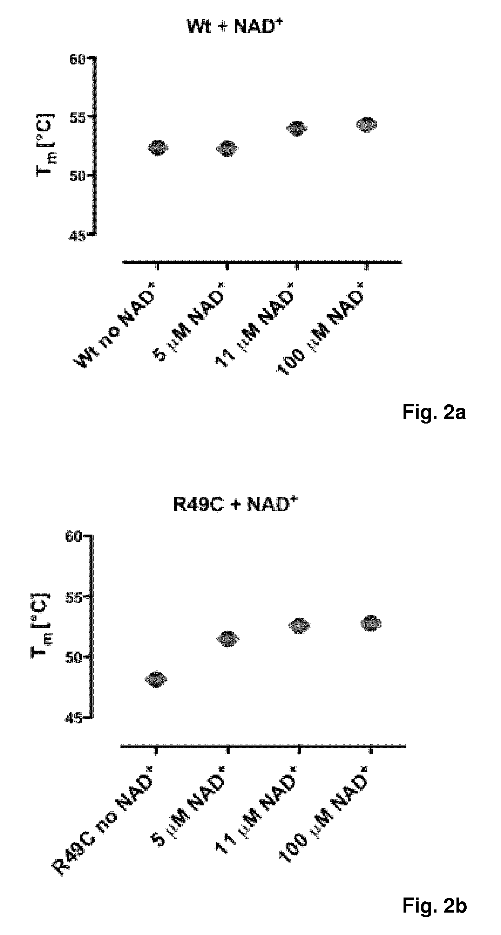

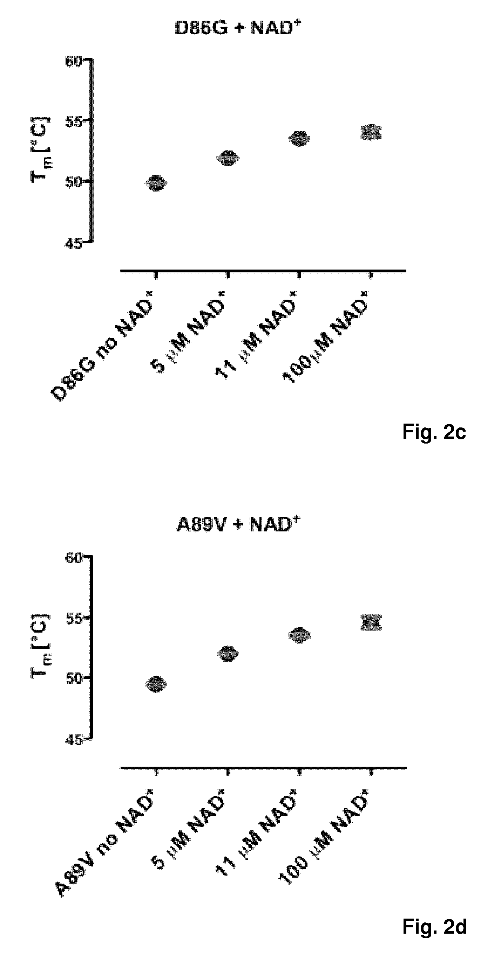

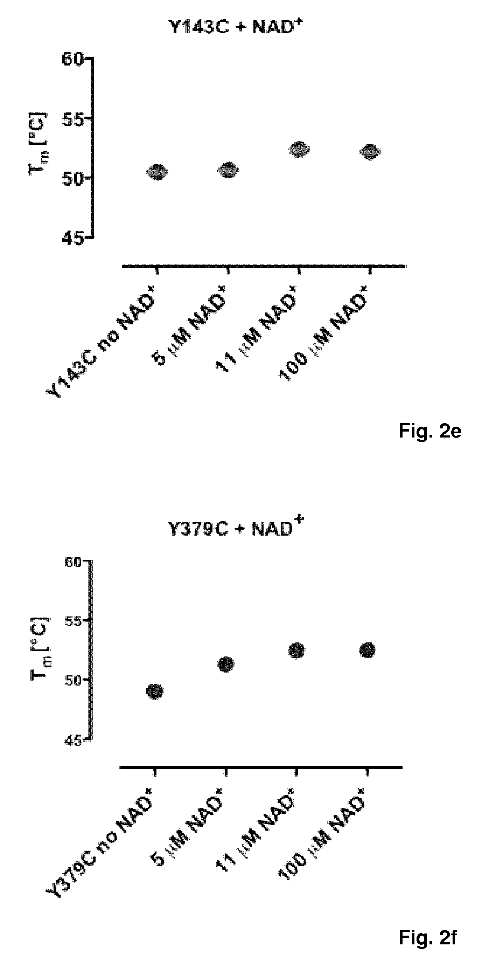

- Fig. 2a - Fig. 2f: show the T_{M} values resulting from the assays according to Fig. 1a - Fig. 1f,
- Fig. 3: shows the T_{M} values obtained with different concentrations of S-NAD⁺,
- Fig. 4: shows the results obtained with different concentrations of E-NAD⁺,
- Fig. 5: shows the results obtained with different concentrations of ribavirin,
- Fig. 6: shows the results obtained with different concentrations of DZNep,
- Fig. 7: shows the results obtained with different concentrations of ilimaquinone and
- Fig. 8a - Fig. 8f: show the results of Right Angle Light Scattering (RALS) measurements obtained for wild-type protein and mutated proteins together with different AHCY binding molecules.

The potential of the binding of NAD⁺ or an analogue of NAD⁺ to the NAD⁺ binding site and of the binding of an analogue of a substrate of AHCY to the substrate binding site of AHCY in stabilizing the enzyme and its activity was analyzed by use of Differential Scanning Fluorimetry (DSF). The following substrates were tested in the given concentrations:

| | Concentration I | Concentration II | Concentration III |
|---|---|---|---|
| NAD⁺ | 5 µM | 11 µM | 100 µM |
| S-NAD⁺ | 5 µM | 11 µM | 100 µM |
| E-NAD⁺ | 5 µM | 11 µM | 100 µM |
| Ribavirin | 1 µM | 10 µM | 100 µM |
| DZNep | 1 µM | 10 µM | 100 µM |
| Ilimaquinone | 1 µM | 10 µM | 100 µM |

The following five mutant proteins were selected for testing:
R49C, D86G, Y143C, A89V and Y379C.

Details of these mutations can be taken from the following table:

| cDNA | Exon | Amino Acid | Mature Protein | Location of Mutation |
|---|---|---|---|---|
| c.145 C>T | 2 | p.Arg49Cys | R49C | β-sheet 1 catalytic domain |
| c.257 A>G | 3 | p.Asp86Gly | D86G | α-Helix 3 catalytic domain |
| c.266 C>T | 3 | p.Ala89Val | A89V | α-Helix 3 catalytic domain |
| c.428 A>G | 4 | p.Tyr143Cys | Y143C | α-Helix 5 catalytic domain |
| c.1139 A>G | 10 | p.Tyr379Cys | Y379C | Connection of α-Helix 19 and β-sheet 15 |

### Expression and purification

E. coli strain DH5a cells (Invitrogen, Thermo Fisher Scientific Inc. USA) were grown in 2YT medium (1% (w/v) tryptone, 1% (w/v) yeast extract, 0.5% (w/v) NaCl) containing 100 µg/ml ampicillin respective 50 µg/ml chloramphenicol for the GroESL encoding DH5a strain. When OD₆₀₀ reached 0.6, IPTG was added/supplemented to a final concentration of 1 mM to induce protein overexpression and culture was continued for an appropriate time (22 h) at various temperatures depending on the expression plasmid. Bacteria were harvested by centrifugation and lysed by sonification. All mutated enzymes were purified from the soluble fractions of E. coli extracts and protein purification was performed using ÄKTAxpress (GE Healthcare USA) at 4 °C by affinity chromatography, followed by size-exclusion chromatography with a HiLoad 16/60 Superdex 200 column (GE Healthcare USA). The fractions containing isolated tetrameric fusion proteins were collected. Protein concentrations were determined using the assay of Bradford.

### Co-overexpression of chaperonins GroESL

Recombinant pMAL-c2E AHCY expression plasmids (New England Biolabs Inc. USA) were co-transformed with pGroESL encoding the proteins GroES and GroEL in E. coli strain DH5a. Except for the supplemental addition of 50 µg/ml chloramphenicol and 100 µg/ml ampicillin to 2YT medium, expression and purification of proteins followed the procedures described above.

### Determination of the thermal stability of AHCY and mutated AHCY

### Differential Scanning Fluorimetry (DSF)

Fluorescence measurements were carried out on a 7900 HT fast real-time PCR System (Applied Biosystems, Thermo Fisher Scientific Inc. USA). The data was recorded in Abi Prism SDS software. Samples contained AHCY-Maltose-Binding Protein fusion proteins, HEPES, and SYPRO Orange fluorescence dye (SYPRO^{®} Orange Protein Gel Stain from Invitrogen, Thermo Fisher Scientific Inc. USA) in a 1:1000 dilution), which binds to exposed hydrophobic sites of unfolded proteins. For this dye, the wavelengths used for excitation and emission are 492 nm, respectively 610 nm. The assay was measured in quadruplicates on a 96-well PCR plate. Thermal denaturation was monitored by following the changes in SYPRO Orange fluorescence emission (excitation at 492 nm, emission at 610 nm). The measurement was performed in a 25 °C to 75 °C range, at a rate of 2 °C/min. Thermal denaturation curves were obtained by the plotting of fluorescence intensities against temperature and T_{M} values as the midpoint of the protein-unfolding transition were calculated using the Boltzmann sigmoidal non-linear regression function in GraphPad Prism (GraphPad Prism 5.0, GraphPad Software Inc. USA). All measurements were corrected for background reduction of SYPRO Orange fluorescence. To analyze the stabilizing effect of different compounds different concentrations of NAD⁺, NAD⁺ analogues and substrate analogues were added to the samples. Significances between wild-type and mutated apoenzymes and holoenzymes with stabilizing agents were calculated by one-way analysis of variance followed by Dunnett's post test (GraphPad Prism 5.0, GraphPad Software Inc. USA).

### Right Angle Light Scattering (RALS)

RALS measurements were performed on a Cary Eclipse fluorescence spectrophotometer (Varian Inc., acquired by Agilent Technologies USA) equipped with a temperature-controlled Peltier multicell holder (Varian Inc., acquired by Agilent Technologies USA). Samples contained AHCY protein (1 mg/ml) in 20 mM HEPES buffer at pH 7.0 containing 200 mM NaCl. The increase in turbidity was monitored in the temperature range from 25 °C to 65 °C at a heating rate of 1 °C/min (excitation at 330 nm, emission 335 nm, 5.0 nm slit width). In the time-dependent assays, temperature was constant and the increase of turbidity was monitored over 60 minutes. Turbidity curves were illustrated with GraphPad Prism 5.0.

### Cell culture

### Eukaryotic expression of AHCY and mutated AHCY

For cell culture experiments the cDNA of human AHCY gene was subcloned into pEF DEST 51 expression vector (Invitrogen, Thermo Fisher Scientific Inc. USA) encoding a c-terminal V5-tag by using the gateway recombination cloning technology (Gateway Cloning Technology, Invitrogen, Thermo Fisher Scientific Inc. USA). Presence and correct orientation of AHCY in the plasmid was initially checked by restriction endonuclease mapping and later verified by DNA sequencing. 2 µg DNA were transfected into 2 million COS-7 cells via electroporation (Amaxa^{™} Nucleofector^{™} Technology, Lonza, Switzerland). Cells were cultured in RPMI 1640 medium with stable glutamine with 10% fetal bovine serum and 1% Gibco® Antibiotic-Antimycotic 100X (Thermo Fisher Scientific Inc. USA). Cells were passaged into fresh growth medium 24 h after transfection. COS cells were harvested by trypsinization, washed with PBS and then collected by centrifugation. Expression of AHCY in transfected COS-7 cells was detected by immunoblotting. Therefore, cells were treated with lysis buffer (50 mM Tris-HCl, pH = 7.4, 150 mM NaCl, and 2 mM complete protease inhibitor) and lysed by three freeze-and-thaw cycles. Total cell lysates were separated on a 4-12% SDS-polyacrylamide gel via electrophoresis and transferred onto a nitrocellulose membrane via electroblot. The membrane was blocked with 5% milk in 1 x TBS (Tris buffered saline, 50 mM Tris [2-Amino-2-hydroxymethyl-propane-1,3-diol] and 150 mM NaCl) followed by 1 h of incubation with the primary antibody, mouse anti-V5 tag (1:5000 dilution, anti-V5 epitope tag antibody R960-25, Thermo Fisher Scientific Inc. USA), and 1 h of incubation with the secondary antibody, anti-mouse HRP (goat anti-mouse IgG (H+L) poly-HRP secondary antibody, Thermo Fisher Scientific Inc. USA). Blots were visualized with a substrate (SuperSignal™ West Femto Chemiluminescent Substrate, Thermo Fisher Scientific Inc. USA) and chemiluminescence was monitored with an imaging system (DIANA III chemiluminescence imager, Raytest USA Inc. USA). Resulting protein bands of interest were quantified by AIDA software (Automatic Image Data Analysis Software, Raytest USA Inc. USA). The amount of target protein is normalized to GAPDH as a reference.

### Statistical analysis

Non-linear regression analyses and statistical tests were performed using GraphPad Prism 4.0c (GraphPad Software Inc. USA).

### Results

### Stabilization against thermal stress

DSF is an excellent means to monitor global thermal unfolding events by probing the accessibility of the fluorescent dye Sypro Orange to hydrophobic groups within a protein. DSF was used in order to assess whether mutated AHCY proteins can be stabilized by various compounds at increasing temperatures. The stabilizing effect was evaluated by comparison of the unfolding transition midpoints (fraction unfolded = 0.5) as an operative measure of protein stability and by graphical analysis of the thermal denaturation curves. The midpoint of thermal unfolding of wild-type AHCY was shifted by an average of 2.27 °C towards higher temperatures by NAD⁺ and NAD⁺ analogues. The addition of substrate analogues had an even more pronounced effect by showing an average shift of 3.86 °C.

A right shift in the unfolding transition was observed for every substance tested, indicating a stabilizing effect on the protein. Among all compounds analyzed, DZNep which addresses the substrate binding site, showed the highest increase in T_{M}, stabilizing the unfolding of D86G by 11.18 °C. Comparable effects were observed for all mutated AHCY proteins.

### Stabilization by the natural cofactor NAD⁺

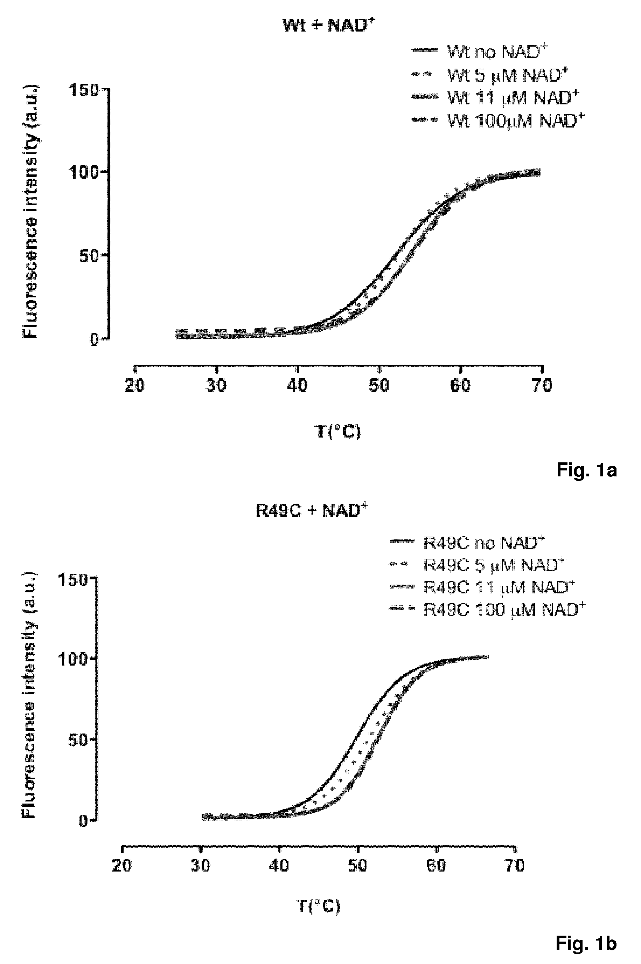

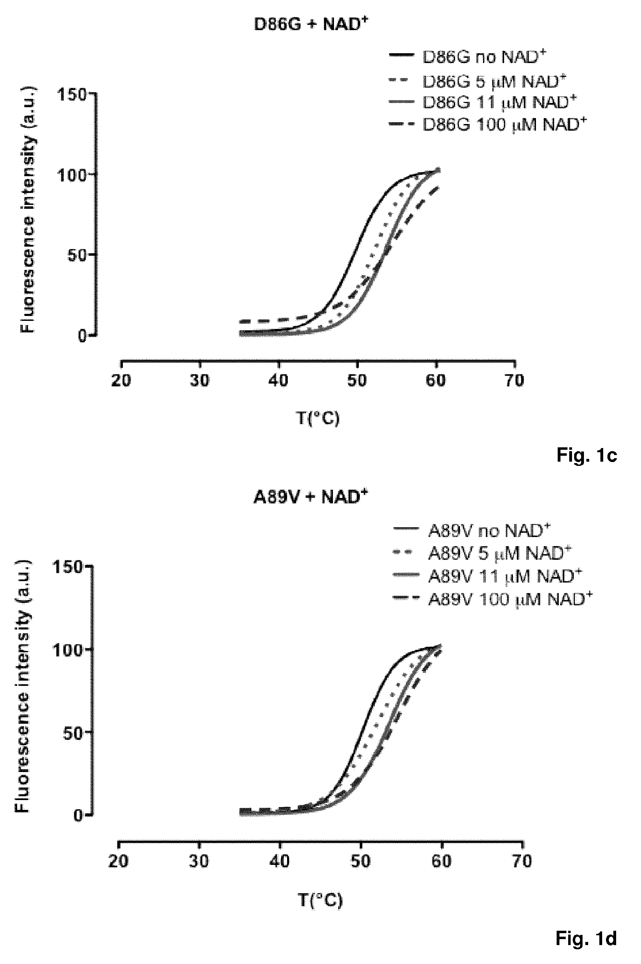

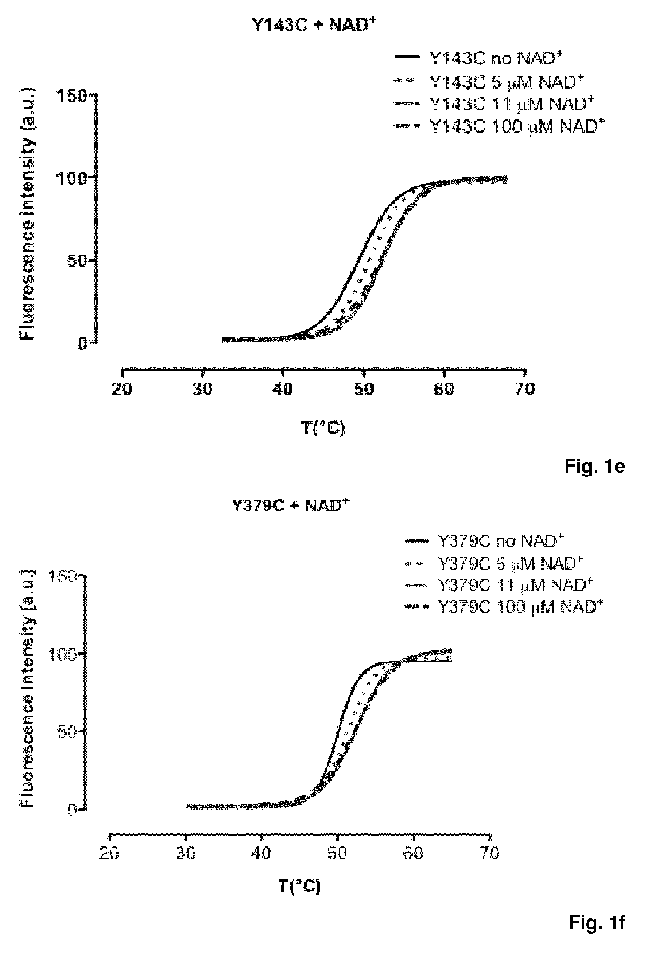

The individual results can be seen from Figs. 1 a to 1f and 2a to 2f and are summarized in the following table:

| Mutation | Wt | | R49C | | D86G | | A89V | | Y143C | | Y379C | |
|---|---|---|---|---|---|---|---|---|---|---|---|---|
| | T_{M} | *SD* | T_{M} | *SD* | T_{M} | *SD* | T_{M} | *SD* | T_{M} | *SD* | T_{M} | *SD* |
| No NAD⁺ | 51,98 | *0,1167* | 49,84 | *0,0802* | 49,55 | *0,0876* | 50,29 | *0,06043* | 49,34 | *0,07141* | 50,01 | *0,0354* |
| NAD⁺ 5 µM | 52,24 | *0,1349* | 51,48 | *0,1213* | 51,88 | *0,0384* | 51,97 | *0,04197* | 50,62 | *0,04564* | 51,31 | *0,0465* |
| NAD⁺ 11 µM | 53,99 | *0,0749* | 52,55 | *0,1219* | 53,51 | *0,035* | 53,52 | *0,16540* | 52,36 | *0,19150* | 52,45 | *0,0689* |
| NAD⁺ 100 µM | 54,32 | *0,2627* | 52,75 | *0,1726* | 54,02 | *0,72* | 54,59 | *0,9564* | 52,17 | *0,07868* | 52,48 | *0,0446* |

| | | | | | | | | | | | | |
|---|---|---|---|---|---|---|---|---|---|---|---|---|
| T_{M} = Temperature at the midpoint of protein unfolding transition *SD* = Standard Deviation | | | | | | | | | | | | |

The cofactor NAD⁺ showed very good effects on the thermal stability of AHCY wild-type and mutated AHCY proteins. For AHCY wild-type, the addition of 100 µM NAD⁺ stabilized the unfolding transition by 2.34 °C. Comparable behavior was observed for the mutated AHCY proteins. D86G showed the highest increase in T_{M} with NAD⁺ (ΔT_{M} = 4.47 °C for 100 µM NAD⁺).

For NAD⁺, the stabilizing effect in the DSF assay was observed to be concentration-dependant.

### Stabilizing effect of the NAD⁺ analogues S-NAD⁺ and E-NAD⁺

S-NAD⁺ is a side-chain thione analogue modified in the nicotinamide part of the coenzyme (Li et al.). E-NAD⁺ (ε-NAD⁺) was initially synthesized by the reaction of chloracetaldehyde with NAD⁺ and is modified in the adenine part of the cofactor.

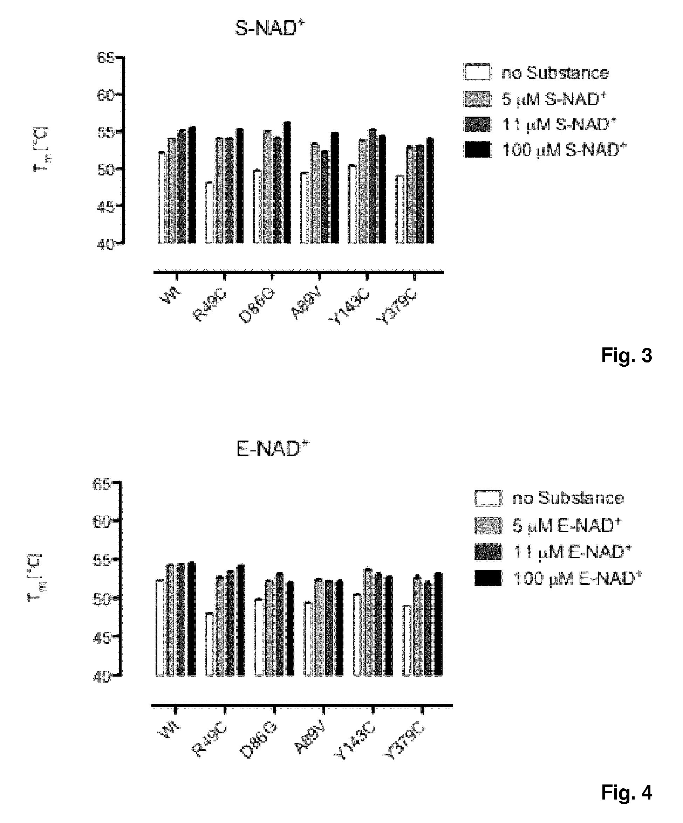

As shown in Fig. 3 S-NAD⁺ showed a similar stabilizing pattern as the natural cofactor NAD⁺. Again, especially the variant D86G showed improved thermal stability: the midpoint of thermal unfolding was shifted by 6.7 °C (100 µM). In summary, upon addition of S-NAD⁺, all mutated AHCY proteins showed improved resistance against thermal stress.

Fig. 4 shows that considerable stabilization was also observed for E-NAD⁺ but the substance was proved to be less effective than S-NAD⁺. Here, the substance was particularly beneficial for the variant R49C, which showed an increase in T_{M} of ΔT_{M} = 4.36 °C.

### Stabilizing effect of ribavirin

Ribavirin is a purine nucleoside analogue, which binds to the adenosine binding site of AHCY. It was described to stop viral RNA synthesis and viral mRNA-capping. Ribavirin is used primarily to treat hepatitis C and viral hemorrhagic fevers.

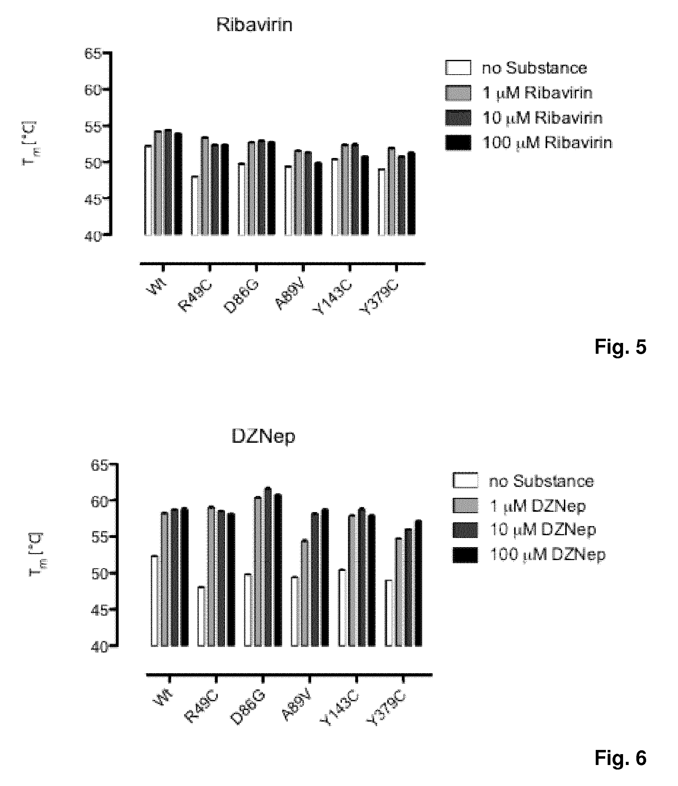

As can be seen from Fig. 5 the addition of ribavirin addressing the substrate binding site showed a good stabilizing effect on AHCY proteins. Both wild-type and mutated AHCY proteins revealed marked changes in the shape of the denaturation curves. The highest increase in T_{M} was shown for R49C. Interestingly, best benefit was observed by adding the substance in a concentration of 1 µM.

### Stabilizing effect of DZNep

DZNep is a competitive inhibitor addressing the substrate binding site of AHCY. As can be seen from Fig. 6 DZNep showed the strongest effect on the thermal shift of all AHCY proteins. Among all mutated AHCY proteins, D86G showed the highest increase of ΔT_{M} (13.5 °C at 10 µM DZNep). A shift of > 4.33 °C was observed for all mutated AHCY proteins. It may be presumed that the responsiveness of AHCY mutants to DZNep is based on the tight binding to the catalytic centre of the enzyme which stabilizes its folding state.

### Stabilizing effect of ilimaquinone

Ilimaquinone is a natural metabolite (sesquiterpene quinone) shown to have antiinflammatory, antimicrobial, and antimitotic properties. As an analogue of adenosine it binds to the active site of AHCY.

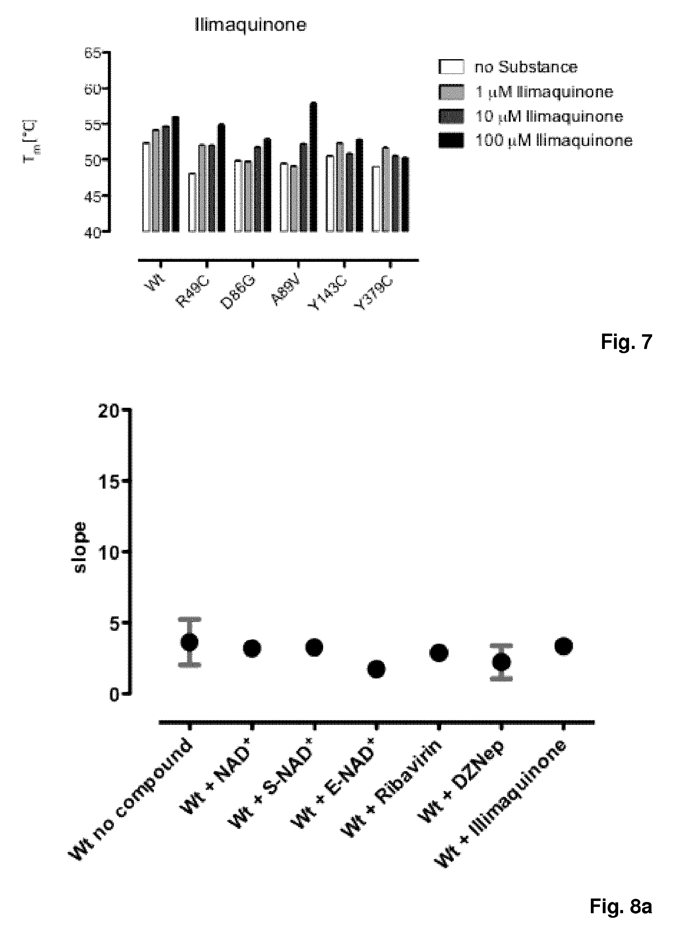

Fig. 7 shows that upon addition of ilimaquinone, a protecting effect against thermal unfolding was revealed. All mutated AHCY proteins showed a benefit concerning the ΔT_{M}. In particular, considerable stabilization was detected for the variant A89V, shifting the midpoint of thermal unfolding by 8.9 °C.

In conclusion, all compounds tested enhanced the thermal stability of their targets.

### Stabilization against protein aggregation

RALS experiments probing the formation of insoluble aggregates were carried out to investigate whether the addition of substances that showed a stabilizing effect in the thermal denaturation assay also prevents early aggregation. For this purpose time-dependant aggregation profiles of wild-type and mutated AHCY proteins were measured at constant temperature of 37 °C.

For evaluation of the data, the slope of the aggregation curve was analyzed by linear regression and statistical significance was determined by one-way analysis of variance using GraphPad Prism (GraphPad Prism 5.0, GraphPad Software Inc. USA).

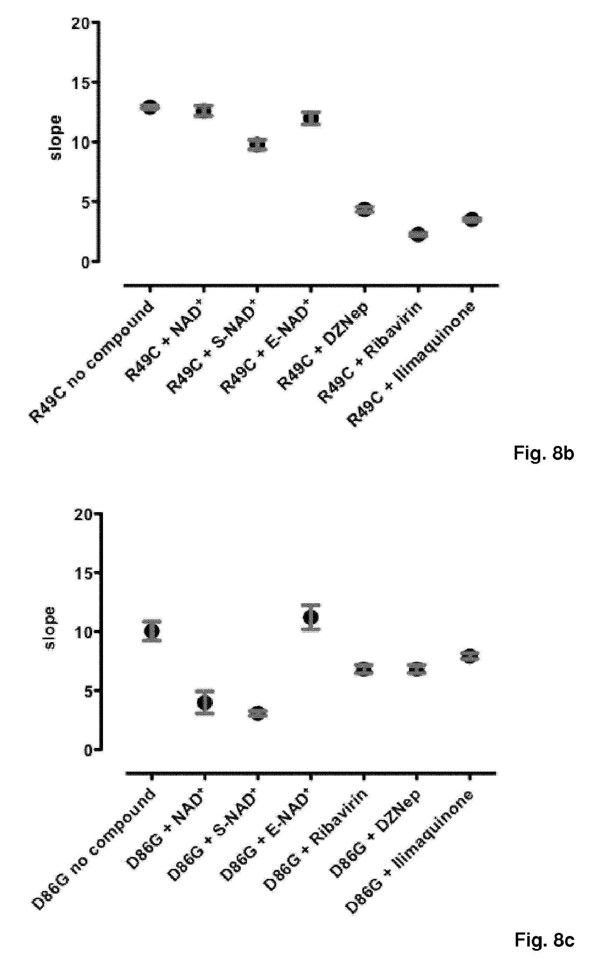

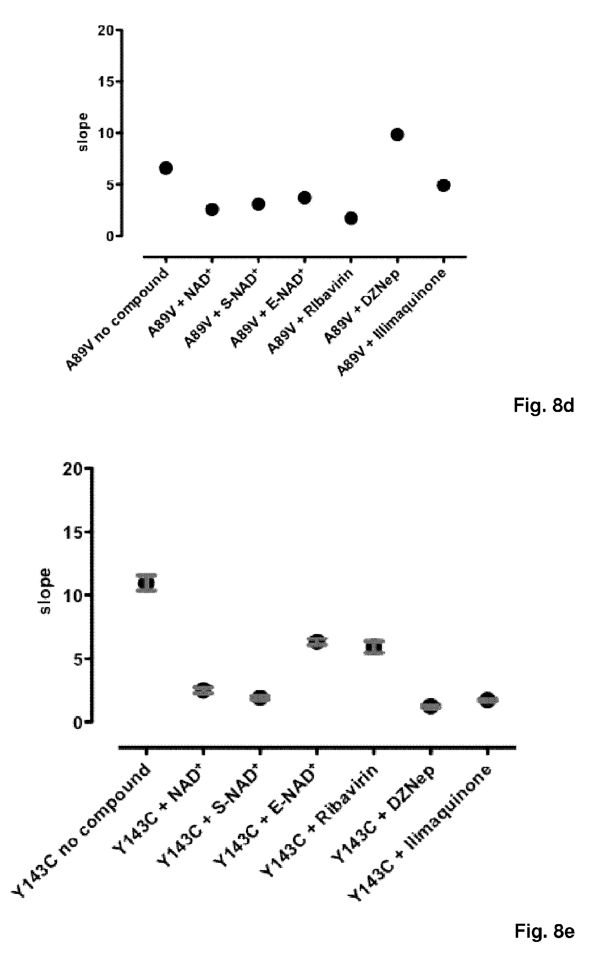

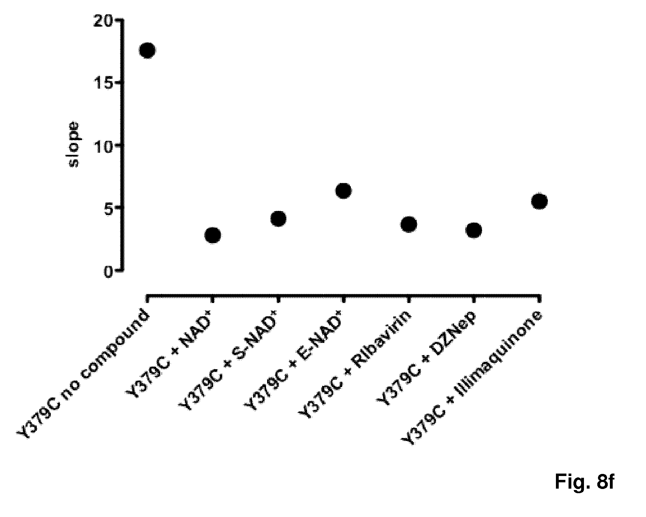

Figures 8a to 8f show that, particularly for mutated AHCY proteins, the binding of small molecules can reduce protein aggregation. Upon addition of NAD⁺, NAD⁺ analogues or substrate analogues, all mutated AHCY proteins showed decelerated aggregation.

## Claims

1. Adenosylhomocysteinase (AHCY) binding substance for use in the treatment of deficiency of function of AHCY in a human being or an animal, wherein the AHCY binding substance is the cofactor nicotinamide adenine dinucleotide (NAD⁺), or an analogue of the cofactor NAD⁺ which analogue binds to an NAD⁺ binding site of AHCY, or an analogue of a substrate of AHCY which analogue binds to a substrate binding site of AHCY.

2. The adenosylhomocysteinase (AHCY) binding substance of claim 1, wherein the analogue of the cofactor NAD⁺ is thionicotinamide adenine dinucleotide (S-NAD⁺), nicotinamide 1,N⁶-ethenoadenine dinucleotide (E-NAD⁺), β-nicotinamide adenine dinucleotide (β-NAD⁺), nicotinamide hypoxanthine dinucleotide (deamino-NAD⁺), nicotinamide guanine dinucleotide, α-dihydronicotinamide adenine dinucleotide (α-NADH), β-dihydronicotinamide adenine dinucleotide (β-NADH), β-nicotinamide adenine dinucleotide phosphate (β-NADP), nicotinamide adenine triphosphate (AP3), ribofuranosyl-nicotinamide diphosphate (dAP2), ribofuranosyl-nicotinamide triphosphate (ApCpp), nicotinamide thymine dinucleotide (TP2), nicotinamide uracil dinucleotide (UP2), nicotinamide cytosine dinucleotide (CP2), nicotinamide guanine dinucleotide (GP2), nicotinamide 8-bromo-adenine dinucleotide (8-Bromo-AP2), 2'-bromo-ribofuranosyl-nicotinamide diphosphate (2'-Bromo-dAP2), P¹-(5'-adenosyl) P³-[5'-(1-β D-ribofuranosyl-nicotinamide)] triphosphate (AP₃(Nic)), 1,2,4-triazole-3-carboxamide adenine dinucleotide (AP₂Ribavirin), P¹-[5'-(2'-deoxyadenosyl)] P²-[5'-(1-β D-ribofuranosyl-nicotinamide)] diphosphate (dAP2(Nic)), P¹-(5'-adenosyl) P³-[5'-(1-β D-ribofuranosyl-nicotinamide)] [(α,β)-methyleno]triphosphate (ApCpp(Nic)), nicotinamide thymine dinucleotide (TP2(Nic)), nicotinamide uracil dinucleotide (UP2(Nic)), nicotinamide cytosine dinucleotide (CP2(Nic)), nicotinamide guanine dinucleotide (GP2(Nic)), nicotinamide 8-bromo-adenine dinucleotide (8-Bromo-AP2(Nic)), an ion of any of said analogues which analogue is not charged, or a hydrate of any of said analogues.

3. The adenosylhomocysteinase (AHCY) binding substance of claim 2, wherein said ion is an ion of a sodium salt.

4. The adenosylhomocysteinase (AHCY) binding substance of claim 1, wherein the analogue of the substrate is 1-[(2*R*,3*R*,4*S*,5*R*)-3,4-dihydroxy-5-(hydroxymethyl)oxolan-2-yl]-1*H*-1,2,4-triazole-3-carboxamide (ribavirin), 3-deazaneplanocin A (DZNep) or ilimaquinone.
